**Europäisches Patentamt**

**European Patent Office**

**Office européen des brevets**

(11) Veröffentlichungsnummer: **0 017 909**
**B1**

(12) **EUROPÄISCHE PATENTSCHRIFT**

(45) Veröffentlichungstag der Patentschrift:
**20.06.84**

(21) Anmeldenummer: **80101897.9**

(22) Anmeldetag: **09.04.80**

(51) Int. Cl.³: **G 01 N 33/54**, C 12 Q 1/40,
G 01 N 31/02

(54) Verfahren zur Bestimmung von Anti-Hyaluronidase und hierfür verwendbares Mittel.

(30) Priorität: **14.04.79 DE 2915309**

(43) Veröffentlichungstag der Anmeldung:
**29.10.80 Patentblatt 80/22**

(45) Bekanntmachung des Hinweises auf die Patenterteilung:
**20.06.84 Patentblatt 84/25**

(84) Benannte Vertragsstaaten:
**AT BE CH DE FR GB IT LI LU NL SE**

(56) Entgegenhaltungen:
CHEMICAL ABSTRACTS, Band 44 , Nr. 21, 10. November 1950, Spalte 10015g-h Columbus, Ohio, U.S.A. T.N. HARRIS et al.: "A comparison of physicochemical measurements of hyaluronidase and neutralizing antibodies thereto"
J. Biol. Chem. Band 252 Heft 2, 1977, S. 420-426
Holloid Zeitschrift Band 181, 1962, S. 52-59

(73) Patentinhaber: **BEHRINGWERKE AKTIENGESELLSCHAFT, Postfach 1140, D-3550 Marburg/Lahn (DE)**

(72) Erfinder: **Toth, Tibor, Dr., Gründeberg 1, D-3550 Marburg/Lahn (DE)**
Erfinder: **Gils, Reiner, Friedrich-Ebert-Strasse 66, D-3578 Schwalmstadt (DE)**

(74) Vertreter: **Schüler, Albert, Dr. et al, HOECHST AKTIENGESELLSCHAFT Zentrale Patentabteilung Postfach 80 03 20, D-6230 Frankfurt am Main 80 (DE)**

## Beschreibung

Die Erfindung betrifft ein Verfahren zur Bestimmung von Anti-Hyaluronidase, das sind Antikörper, die gegen Hyaluronidase gerichtet sind, und das hierfür verwendbare diagnostische Mittel.

Hyaluronidase ist ein extrazelluläres Stoffwechselprodukt von Streptokokken der Gruppe A nach Lancefild. Die Hyaluronidase ist ein Antigen, das im Menschen die Bildung spezifischer Antikörper induziert. Die Kenntnis der Konzentration der Antikörper im menschlichen Blut ist von diagnostischer Bedeutung.

Bei dem von Streptokokken verursachten rheumatischen Fieber stellt man neben einem Anstieg des Antistreptolysin-Titers häufig auch einen Anti-Hyaluronidaseanstieg im Blut der Patienten fest. Zumeist besteht zwischen diesen Werten eine gute Übereinstimmung, doch zeigen etwa 20% der Patienten mit Streptokokken-Infektionen der Gruppe A keine signifikante Erhöhung der Anti-Streptolysin-O-Titer, jedoch einen deutlich erhönten Anti-Hyaluronidase-Titer. Es besteht deshalb ein Bedürfnis für eine diagnostische Bestimmung der Anti-Hyaluronidase.

Es wurde eine grosse Anzahl von Methoden zur Bestimmung von Anti-Hyaluronidase beschrieben. Die wichtigsten sind:

1. Mucingerinsel-Verminderungstest nach Quinn (R.W. Quinn, J. clin. Invest. 27, 463 (1948));

2. Trübungsverminderungstest nach Faber (V. Faber, Acta path. Microbiol. Scand. 31, 345 (1952));

3. Viskositäts-Minderungstest nach Quibell (J. Madinaveitia und T.H.H. Quibell, Biochem. J. 34, 625 (1940)).

Bei der Methode nach Quinn wird die Hemmung der Hyaluronidase, die spezifisch Hyaluronsauren abbaut (saure, aus N-Acetylglucosamin und Glucuronsaure aufgebaute Mucopolysaccharide) bestimmt. Das Vorhandensein von Antikörpern gegen Hyaluronidase wird durch die Aufhebung der in Gegenwart von Hyaluronidase auftretenden Hemmung der Hyaluronsaure-Gerinnung, die bei saurem pH-Wert einsetzt, angezeigt.

Der Trübungsverminderungstest und seine Modifikationen nutzen die Aufhebung der in Gegenwart von Hyaluronidase auftretenden Hemmung der Trübung von Hyaluronsäure-Lösung durch Anti-Hyaluronidase.

Bei dem Viskositäts-Minderungstest wird die Aufhebung der in Gegenwart von Hyaluronidase auftretenden Herabsetzung der Viskosität von Hyaluronsaure-Lösung durch Anti-Hyaluronidase gemessen.

Diese Methoden haben eine Reihe von Nachteilen für diagnostische Laboratorien. (Vgl. Bergmeier, Methoden der enzymatischen Analyse, Band I, S. 984).

Der Mucingerinsel-Verminderungstest ist wenig empfindlich und erfordert grosse Mengen Enzym für die Durchführung des Testes. In Mikrotiter-Platten wird der Test ungenau.

Die Trübungsmessung ist nur für gereinigte Hyaluronidasen geeignet und benötigt ein besonders sauberes Substrat. Sie muss in Details sehr sorgfältig durchgeführt werden und erfordert grossen apparativen Aufwand (Photometer).

Die Ergebnisse der viskosimetrischen Verfahren hängen von der Reinheit des Substrates ab; die Abnahme der Viskosität aufgrund nichtenzymatischer Reaktionen ist ein weiteres Problem.

Andere Methoden sind zu unempfindlich oder benötigen grosse Mengen Enzym für die Durchführung des Testes.

In Kolloid-Zeitschrift Band 81, 1962, Seiten 52 – 59 wurde bereits über das Fällungsverhalten von Hyaluronsäure gegenüber Cetyltrimethylammoniumbromid berichtet. Aus J. Biol. Chem. Band 252, Heft 2, 1977, Seiten 420 – 426 geht hervor, dass Hyaluronsäure mit Cetylpyridiniumchlorid gefällt werden kann.

Es wurde nun ein Verfahren gefunden, das die Bestimmung des Anti-Hyaluronidase-Titers mit hoher Empfindlichkeit und grosser Spezifität ermöglicht. Ein weiterer Vorteil der Methode ist ihre schnelle Durchführbarkeit.

Gegenstand der Erfindung ist ein Verfahren zur Bestimmung der Anti-Hyaluronidase in wässriger Lösung in einem Reihenverdünnungstest mittels einer definierten Menge Streptokokken-Hyaluronidase und danach einer definierten Menge Hyaluronsäure, Ausfällen der durch Streptokokken-Hyaluronidase nicht abgebauten Hyaluronsäure und Bestimmen der Fällungsgrenzkonzentration, dadurch gekennzeichnet, dass das Fällungsmittel ein Komplex aus einem kationischen Detergenz aus der Gruppe der quaternären Ammoniumverbindungen und einem Dis-Azofarbstoff ist, wobei der Komplex auf 10 Gewichtsteile Detergenz 1 – 10 Gewichtsteile Farbstoff enthält.

Das Verfahren beruht auf der Erkenntnis, dass die durch Hyaluronidase nicht abgebaute Hyaluronsäure mit dem Fällungsmittel-Farbstoffkomplex ausgefällt werden kann unter Bildung eines farbigen Präzipitates, das sehr gut sichtbar ist.

Der wichtigste Bestandteil des Verfahrens ist der Fällungsmittel-Farbstoffkomplex.

Als kationische Detergentien aus der Gruppe der quaternären Ammoniumverbindungen eignen sich solche wie Cetyltrimethylammoniumhalogenid, vor allem -bromid oder -chlorid, oder Pyridiniumverbindungen wie Cetylpyridinium-halogenide, vor allem das Chlorid. Bevorzugt wird jedoch das Cetyltrimethylammoniumbromid.

Als Dis-Azofarbstoffe ist vor allem Kongorot geeignet.

Durch Verwenden von Farbstoffmischungen kann die Ablesbarkeit der Fällungsgrenze noch verbessert werden. Verwendet man z. B. Toluidinblau-Lösung, so erhält man ein orangerotes Präzipitat auf oder in hellblauem Untergrund. Zur Verbesserung der Ablesbarkeit können aber auch andere Farbstoffe wie z. B. Methylgrün oder Bromphenolblau verwendet werden. Besonders gut geeignet zur Bildung eines blauen Untergrunds ist Toluidinblau.

Der erfindungsgemässe Fällungsmittel-Farbstoffkomplex setzt sich aus folgendem Verhältnis der Bestandteile zusammen: Auf 10 Gewichtsteile des kationischen Detergenzes aus der Gruppe quarternärer Ammoniumverbindungen werden 1 – 10 Gewichtsteile des Dis-Azofarbstoffes zugesetzt. Die Vermischung der beiden ggf. auch mehrerer Komponenten erfolgt in wässriger Lösung bei 20 – 100°C Temperatur, vorzugsweise 60 – 70°C.

Ein für die Ausübung der Erfindung geeignetes Hyaluronsäure-Substrat kann beispielsweise wie folgt hergestellt werden: 1 Gewichtsteil Hyaluronsäure wird mit 50 – 250 Gewichtsteilen einer Aminosäure, z.B. Glycin oder ß-Alanin in 1000 ml Wasser suspendiert. Die Mischung wird bis zum vollständigen Lösen der Hyaluronsäure bei 0 – 50°C stehengelassen, anschliessend filtriert und lyophilisiert. Mannit oder Albumin sind als Lyophilisationszusätze ebenfalls geeignet.

Zur Herstellung des Fällungsmittel-Farbstoffkomplexes kann beispielsweise wie folgt vorgegangen werden:

1 Gewichtsteil Cetyltrimethylammoniumbromid (CTA) und 1 – 2 Gewichtsteile NaOH werden in einem Erlenmeyerkolben mit destilliertem Wasser suspendiert und unter ständigem Schwenken auf 60 – 70°C erwärmt. Anschliessend wird die Lösung auf Raumtemperatur abgekühlt und der pH-Wert der Lösung mit Essigsäure auf 3,5 – 10 eingestellt. Nach Filtrieren werden zu einem Volumenteil der Lösung unter Rühren 2 – 10 Volumenteile Kongorot-Lösung evtl. 0,01 – 0,5 Volumenteile 0,1 M Toluidinblau-Lösung und 20 – 40 Volumenteile destilliertes Wasser gegeben.

Gegenstand der Erfindung ist auch der Fällungsmittel-Farbstoffkomplex als solcher, gekennzeichnet durch einen Gehalt an 10 Gewichtsteilen eines kationischen Detergenzes aus der Gruppe quarternärer Ammoniumverbindungen und 1 – 10 Gewichtsteilen, vorzugsweise 4 Gewichtsteilen, eines Dis-Azofarbstoffes, vor allem Cetyltrimethylammoniumhalogenid und dem Dis-Azofarbstoff Kongorot, vorzugsweise in wässriger Lösung oder auch in lyophilisierter Form.

Das erfindungsgemässe Verfahren ist wegen seiner Einfachheit für Serienanalysen geeignet. Zweckmässig wird hierfür ein sogenannter Testkit zusammengestellt, der aus folgenden Reagentien besteht:

1. Hyaluronsäure-Substrat, bestehend im wesentlichen aus Hyaluronsäure und Glycin, trocken oder in Lösung (0,35 mg Hyaluronsäure, 50 mg Glycin in 1 m destilliertem Wasser). Für das Hyaluronsäure-Substrat sind die im Handel erhältlichen Hyaluronsäuren oder ihre Salze brauchbar.

2. Streptokokken-Hyaluronidase, z.B. eine Enzympräparation aus dem Kulturfiltrat von Streptokokken der Gruppe A, hergestellt nach L.W. Wannamaker und W. Yasmineh, J. Exp. Med. 126 (1967) 475.

Die Lösung ist mit einer Aktivität von 20 IU/ml bis 300 IU/ml, vorzugsweise etwa 75 – 150 IU/ml gebrauchsfertig. Die im Handel erhältlichen Hyaluronidasen sind für den Test kaum geeignet, da der Abbau von Hyaluronsäure nur bei Hyaluronidasen durch Anti-Hyaluronidase im Blut gehemmt wird, die aus Streptokokken gewonnen werden.

Bevorzugt wird Streptokokken-Hyaluronidase aus dem Kulturfiltrat von Streptokokken der Gruppe A.

3. Farbstoff-Reagenz, bestehend aus dem Fällungsmittel-Farbstoffkomplex in wässriger Lösung. In der bevorzugten Ausführungsform ist dies Cetyltrimethylammoniumbromid und Kongorot im Verhältnis 10 zu 4. Der Komplex liegt in der wässrigen Lösung in einer Konzentration von 1 g bis 2 g/Liter, vorzugsweise etwa 1,4 g/Liter vor.

4. Anti-Streptokokken-Hyaluronidase-Standard ist ein Humanserum mit einer definierten Konzentration von Streptokokken-Anti-Hyaluronidase, das bei vorschriftsmässiger Durchführung des Tests einen Titer von 256 ergibt.

Das Verfahren wird bevorzugt in Reihenanalysen durchgeführt, wobei Verdünnungsreihen der Lösungen, in denen die Konzentration der Anti-Hyaluronidase bestimmt werden soll, angelegt werden. Wird Serum verwendet, ist es zweckmässig, dieses kurzzeitig auf etwa 56°C zu erwärmen. Dabei werden unspezifische Reaktionen, die das Messergebnis verfälschen könnten, ausgeschaltet. Zu jeder Verdünnung der Probe wird eine bekannte Menge der Hyaluronidase gegeben, die man mit der Anti-Hyaluronidase reagieren lässt. Danach wird die Probe mit Hyaluronsäure versetzt, auf welche nur die nicht durch Anti-Hyaluronidase abgebundene Hyaluronidase einwirken kann. Nicht durch Hyaluronidase abgebaute Hyaluronsäure wird schliesslich mit dem Fällungsmittel-Farbstoffkomplex ausgefällt. Die jeweiligen Verdünnungsreihen werden mit Standardreihen der Anti-Hyaluronidase und der Hyaluronsäure in Beziehung gesetzt.

Für die Durchführung der Reaktionen ist eine Mikrotiterplatte besonders geeignet, da hierauf neben einer Reihe von Seren eine Standardreihe für die Anti-Hydraluronidase sowie entsprechende Kontrollen für das Enzym und das Substrat nebeneinander aufgetragen werden können.

Das folgende Beispiel erläutert die Erfindung:

Beispiel:
1. Vorbereitung der Reagentien
1.1. Hyaluronsäure (1,7 mg), lypohilisiert, wird in 5 ml destilliertem Wasser aufgenommen.
1.2. Streptokokken-Hyaluronidase (4,3 mg), lyophilisiert, wird in 3 ml destilliertem Wasser gelöst.
1.3. Cetyltrimethylammoniumbromid-Farbstoffkomplex ist eine wässrige Lösung von 5,7 mg Cetyl-trimethylammoniumbromid und 2,1 mg Kongorot in 5 ml Wasser.
1.4. Anti-Streptokokken-Hyaluronidase-Standard-Serum ist ein flüssiges, stabilisiertes Humanserum, das bei vorschriftsmässiger Testdurchführung einen Anti-Hyaluronidase-Titer von 300 besitzt.
2. Herstellung der Serum-Verdünnungsreihe
1.5. Jedes zu untersuchende Serum wird vorher 30 min in einem Wasserbad bei 56°C gehalten. Hierfür wird eine 1:16 Verdünnung des Patientenserums verwendet, das ist 0,1 ml Serum + 1,5 ml destilliertes Wasser.
1.6. Anti-Hyaluronidase-Standard-Serum ebenso 1:16 verdünnen.

1.7. Eine Testplatte mit mehreren Reihen von Vertiefungen und mindestens 8 Vertiefungen in einer Reihe wird beschriftet zur Kennzeichnung der verschiedenen zu testenden Serumproben sowie der anzulegenden Serumverdünnung.

1.8. In jede Vertiefung einer jeden für die Serum- bzw. Standardverdünnung vorgesehenen Reihe sowie in der Reihe der Enzymkontrolle werden 35 µl destilliertes Wasser pipettiert. In die Vertiefung für die Substrat-Kontrolle werden 5o µl destilliertes Wasser eingefüllt.

1.9. In die erste Vertiefung der Serumreihen werden 25 µl der 1 : 16 Serum-Verdünnung pipettiert, in eine weitere Reihe die 1 : 16 Verdünnung des Standards.

1.10. Mit Hilfe einer 25 µl abgebenden Mikroverdünner-Pipette werden Verdünnungsreihen mit einem Endvolumen von 25 µl in Zweierstufen, beginnend mit 1 : 16 bis 1 : 4096 hergestellt.

Testansatz

Jede Vertiefung der Verdünnungsreihen (Standard- und Patientensera) enthält zunächst 25 µl einer Serumverdünnungsstufe.

1.11. Zu jeder Vertiefung (ausser Substratkontrolle) werden 25 µl Streptokokken-Hyaluronidase-Lösung mit einer Mikropipette hinzugegeben. Nach dem Mischen mit einem Schüttelgerät wird die Testplatte abgedeckt und mindestens 15 min bei Raumtemperatur stehengelassen.

1.12. Anschliessend wird in alle Vertiefungen (auch für die Enzym- und Substratkontrolle) 25 µl Hyaluronsäure-Substrat-Lösung eingefüllt, gut gemischt, die Platte abgedeckt und 20 min. bei 37°C stehengelassen.

1.13. Danach wird jede Vertiefung 50 µl Cetyltrimethylammoniumbromid-Kongorot-Komplex pipettiert und mit einem Vibrationsgerät 30 Sekunden geschüttelt. Abschliessend wird die Grenzkonzentration der Ausfällung visuell bestimmt. Man kann, falls man eine geeignete Mikrotiterplatte für die Bestimmung verwendet hat, hier auch kurzzeitig zentrifugieren und die Ablesung danach vornehmen.

Bewertung

Zum Ablesen des Ergebnisses werden die Platten zweckmässig auf eine Milchglasscheibe, die von unten beleuchtet werden kann, gestellt oder auch auf einen Ablesespiegel. Es werden diejenigen Verdünnungsstufen bei Standard- und Patientenseren notiert, bei denen eine orangefarbene Präzipitation noch sichtbar wahrgenommen werden kann.

Der Anti-Hyaluronidase-Titer ergibt sich durch Multiplikation von Verdünnungsfaktor des Patientenserums mit der Anti-Hyaluronidase-Konzentration im Standardserum, das z. B. in der Titerstufe 1 : 256 die Fällungsgrenze zeigt, dividiert durch den Verdünnungsfaktor im Standard-Serum.

Z. B. Standard-Serum, Präzipitat in der Verdünnung 1 : 512 Patienten-Serum, Präzipitat in der Verdünnung 1 : 1024 ergibt.

$$\frac{1024 \times 256}{512}$$ = 512 als Titer der Anti-Hyaluronidase im Patientenserum.

In einem Vergleichsversuch, in dem statt des erwähnten Farbstoffkomplexes in Ziffer 1.13 Cetyltrimethylammoniumbromid-Lösung zu dem Testansatz pipettiert wird, kann eine Präzipitation schon in der Verdünnung von 1 : 16 nicht mehr erkannt werden, während mit dem Fällungsmittel-Farbstoff-komplex das Präzipitat noch in einer Verdünnung von 1 : 256 nachgewiesen werden kann.

**Patentansprüche**

1. Verfahren zur Bestimmung der Anti-Hyaluronidase in wässriger Lösung in einem Reihenverdünnungstest mittels einer definierten Menge Streptokokken-Hyaluronidase und danach einer definierten Menge Hyaluronsäure, Ausfällen der durch Streptokokken-Hyaluronidase nicht abgebauten Hyaluronsäure und Bestimmen der Fällungsgrenzkonzentration, dadurch gekennzeichnet, dass das Fällungsmittel ein Komplex aus einem kationischen Detergenz aus der Gruppe der quaternären Ammoniumverbindungen und einem Dis-Azofarbstoff ist, wobei der Komplex auf 10 Gewichtsteile Detergenz 1 – 10 Gewichtsteile Farbstoff enthält.

2. Verfahren nach Anspruch 1, dadurch gekennzeichnet, dass der Fällungsmittel-Farbkomplex aus einem Cetyltrimethylammoniumhalogenid und einem Dis-Azofarbstoff besteht.

3. Verfahren nach einem der Ansprüche 1 und 2, dadurch gekennzeichnet, dass der Fällungsmittel-Farbstoffkomplex aus Cetyltrimethylammoniumbromid oder -chlorid und Kongorot besteht.

4. Mittel zur Fällung von Hyaluronsäure, gekennzeichnet durch einen Gehalt an einem Komplex aus 10 Gewichtsteilen eines kationischen Detergenzes aus der Gruppe der quaternären Ammoniumverbindungen und 1 – 10 Gewichtsteilen eines Dis-Azofarbstoffes.

5. Mittel zur Fällung von Hyaluronsäure, gekennzeichnet durch einen Gehalt von 10 Gewichtsteilen Cetyltrimethylammoniumhalogenid und 4 Gewichtsteilen eines Dis-Azofarbstoffs.

6. Mittel zur Fällung von Hyaluronsäure, gekennzeichnet durch einen Gehalt von 10 Gewichtsteilen Cetyltrimethylammoniumchlorid oder -bromid und 4 Gewichtsteilen Kongorot.

7. Mittel nach einem der Ansprüche 4 bis 6, dadurch gekennzeichnet, dass es ausserdem noch Toluidinblau enthält.

**Claims**

1. Process for determining anti-hyaluronidase in an aqueous solution in a series dilution test by adding a defined amount of streptococcal hyaluronidase and thereafter a defined amount of hyaluronic acid, precipitating the hyaluronic acid not degraded by the streptococcal hyaluronidase and determining the limiting concentration at which a precipitate forms, which comprises that precipitation is effected by a complex of a cationic detergent out of the group of quarternary ammonium compounds and a dis-azo dyestuff, whereby the complex contains 1 to 10 parts weight of dyestuff per 10 parts by weight of detergent.

2. Process as claimed in claim 1 wherein the precipitant-dyestuff complex consists essentially of a cetyl trimethyl ammonium halide and a dis-azo dyestuff.

3. Process as claimed in either one of claims 1 and 2, wherein the precipitant-dyestuff complex consists essentially of cetyl trimethyl ammonium bromide or chloride and Congo red.

4. Agent for the precipitation of hyaluronic acid, which contains a complex of 10 parts by weight of a cationic detergent out of the group of quaternary ammonium compounds and from 1 to 10 parts by weight of dis-azo dyestuff.

5. Agent for the precipitation of hyaluronic acid, which contains 10 parts by weight of a cetyl trimethyl ammonium halide and 4 parts by weight of a dis-azo dyestuff.

6. Agent for the precipitation of hyaluronic acid, which contains 10 parts by weight of cetyl trimethyl ammonium chloride or bromide and 4 parts by weight of Congo red.

7. Agent as claimed in any one of claims 4 to 6 which additionally contains toluidine blue.

**Revendications**

1. Procédé pour la détermination de l'anti-hyaluronidase en solution aqueuse dans un test de dilution en série au moyen d'une quantité définie de streptocoque-hyaluronidase, puis d'une quantité définie d'acide hyaluronique, par précipitation de l'acide hyaluronique non dégradé par la streptocoque-hyaluronidase et détermination de la concentration limite de précipitation, lequel procédé est caractérisé en ce que l'agent de précipitation est un complexe d'un détergent cationique du groupe des composés d'ammonium quaternaires et d'un colorant diazoïque, le complexe contenant de 1 à 10 parties en poids de colorant pour 10 parties en poids de détergent.

2. Procédé selon la revendication 1, caractérisé en ce que le complexe agent de précipitation-colorant est constitué d'un halogénure de cétyltriméthylammonium et d'un colorant diazoïque.

3. Procédé selon l'une quelconque des revendications 1 et 2, caractérisé en ce que le complexe agent de précipitation-colorant est constitué de bromure ou de chlorure de cétyltriméthylammonium et de rouge Congo.

4. Agent pour la précipitation de l'acide hyaluronique, caractérisé par une teneur en un complexe composé de 10 parties en poids d'un détergent cationique du groupe des composés d'ammonium quaternaires et de 1 à 10 parties en poids d'un colorant diazoïque.

5. Agent pour la précipitation de l'acide hyaluronique, caractérisé par une teneur de 10 parties en poids d'halogénure de cétyltriméthylammonium et de 4 parties en poids d'un colorant diazoïque.

6. Agent pour la précipitation de l'acide hyaluronique, caractérisé par une teneur de 10 parties en poids de chlorure ou de bromure de céthyltriméthylammonium et de 4 parties en poids de rouge Congo.

7. Agent selon l'une quelconque des revendications 4 à 6, caractérisé en ce qu'il contient en outre encore du bleu de toludine.